# EUROPÄISCHE PATENTANMELDUNG

(11) **EP 4 464 357 A1**
(43) Veröffentlichungstag der Anmeldung: **20.11.2024**
(21) Anmeldenummer: 24000052.1
(22) Anmeldetag: 13.05.2024
(51) Int. Cl.: A61M 16/04, A61M 16/10

(54) **FILTERSCHUTZHALSTUCH**

(30) Priorität: 19.05.2023 DE 202023001112 U
(71) Anmelder: Tlusteck, Oliver, 99880 Waltershausen (DE)
(72) Erfinder: Tlusteck, Oliver, 99880 Waltershausen (DE)
(74) Vertreter: Weihrauch, Frank

(57) **Zusammenfassung**

Die Erfindung betrifft ein Filterschutzhalstuch, aufweisend einen Überdeckungsabschnitt (1), einen ersten Anlageabschnitt (2), eine erste Luftdurchlassfläche (25), einen zweiten Anlageabschnitt (3) und eine zweite Luftdurchlassfläche (35),wobei der Überdeckungsabschnitt (1) an einer Halstuchvorderseite angeordnet ist, eine außenseitig gerichtete Ausformung (11) aufweist und innenseitig einen Filteraufnahmeraum (4) ausbildet, wobei die Ausformung (11) zur außenseitigen Überdeckung und der Filteraufnahmeraum (14) zur Aufnahme eines Tracheostomafilters ausgebildet ist, wobei der Überdeckungsabschnitt (1) jeweils distal einen vertikalen ersten und einen zweiten Überdeckungsabschnittsrand (12, 13) aufweist, wobei der erste Anlageabschnitt (2) distal zu dem Überdeckungsabschnitt (1) angeordnet und innenseitig für eine flächige Anlage am Hals eines Anwenders ausgebildet ist sowie an einem proximalen ersten Anlageabschnittsende (21) einen ersten Anlageabschnittsrand (22) und an einem distalen ersten Anlageabschnittsende (23) ein erstes Verschlussmittel (24) aufweist, wobei die erste Luftdurchlassfläche (25) durch eine zwischen dem ersten Überdeckungsabschnittsrand (12) und dem ersten Anlageabschnittsrand (22) aufgespannte Fläche gebildet ist, wobei der zweite Anlageabschnitt (3) distal zu dem Überdeckungsabschnitt (1) angeordnet und innenseitig für eine flächige Anlage am Hals eines Anwenders ausgebildet ist sowie an einem proximalen zweiten Anlageabschnittsende (31) einen zweiten Anlageabschnittsrand (32) und an einem distalen zweiten Anlageabschnittsende (33) ein zweites Verschlussmittel (34) aufweist, wobei die Verschlussmittel (24, 34) zusammen einen nackenseitigen Verschluss (4) ausbilden, wobei die zweite Luftdurchlassfläche (35) durch eine zwischen dem zweiten Überdeckungsabschnittsrand (13) und dem zweiten Anlageabschnittsrand (32) aufgespannte Fläche gebildet ist.

## Beschreibung

Die Erfindung betrifft ein Halstuch für Träger eines Tracheostomatubus mit einem außenliegenden Filter mit einer Schutzfunktion für den Filter.

Aus dem Stand der Technik ist es bekannt, Tracheostomafilter mittels eines Verbandes am Hals des Trägers zu fixieren und so gegen mechanische Einwirkungen zu schützen. Das Anlegen eines solchen Verbandes ist zeitlich aufwändig und erfordert entweder die Ausführung durch medizinisches Personal oder bei Ausführung durch den Träger eine ausreichende Schulung und Beweglichkeit des Trägers. Zudem kann die deutliche optische Wahrnehmbarkeit den Träger in seinem sozialen Leben belasten.

Die Aufgabe der Erfindung ist es, ein Hilfsmittel für Träger eines Tracheostomatubus mit einem außenliegenden Filter bereitzustellen, das den Filter vor mechanischen Einflüssen und sonstigen Umgebungseinflüssen wie Wind, Regen, Verschmutzung und Kälte schützt, optisch unauffällig und einfach anwendbar ist und einen hohen hygienischen Standard aufweist sowie kostengünstig herstellbar ist.

Die Aufgabe wird durch die im Patentanspruch 1 aufgeführten Merkmale gelöst. Bevorzugte Weiterbildungen ergeben sich aus den Unteransprüchen.

Dem erfindungsgemäßen Filterschutzhalstuch liegt insbesondere Folgendes zu Grunde.

Als medizinische Maßnahme kann es zur Sicherung der Atmung, insbesondere auch bei Klein- und Kleinstkindern erforderlich sein, mittels Tracheotomie chirurgisch im Halsbereich einen Zugang zur Luftröhre zu schaffen. In diesen Zugang, bezeichnet als Tracheostoma, wird ein Tubus eingesetzt, der nach außen führt und einen ausreichenden Luftführungsquerschnitt bereitstellt. Nachfolgend wird ein solcher Tubus als Tracheostomatubus bezeichnet. Um das Einatmen von Staub und Fremdkörpern zu vermeiden, die Einatemluft zu reinigen, zu temperieren und zu befeuchten ist es bekannt, einen Filter auf den außenseitigen Ausgang des Tubus zu setzen. Die Ausatemluft durchströmt den Filter, wobei ein Filtereinsatz vorzugsweise sowohl Wärme als auch Feuchtigkeit der Ausatemluft speichern kann, die dann wieder an die Einatemluft abgegeben wird. Solche Filter werden auch als HME-Filter oder künstliche Nasen bezeichnet. Sie können insbesondere als längsseitige Filter ausgebildet sein und eine T-Form aufweisen, wobei der Mittelsteg luftführend mit dem Tubus verbunden ist und die beiden Enden des quer liegenden Rohrabschnitts jeweils einen Filtereinsatz aufnehmen. Bei bestimmungsgemäßer Anwendung liegt der querliegende Rohrabschnitt am Hals des Trägers an und die an den beiden Enden angeordneten Öffnungen sind nach links und rechts gerichtet. Durch die Öffnungen wird die Ausatemluft ausgeleitet und die Einatemluft aufgenommen. Nachfolgend wird ein solcher Filter als Tracheostomafilter bezeichnet.

Das erfindungsgemäße Filterschutzhalstuch überdeckt mit einem Überdeckungsabschnitt den Tracheostomafilter und sichert über eine erste und eine zweite Luftdurchlassfläche die ungehinderte Ausleitung der Ausatemluft und die ungehinderte Einleitung der Einatmenluft.

Im Einzelnen liegt wird dies dadurch ermöglicht, dass das Filterschutzhalstuch als Hauptkomponenten einen Überdeckungsabschnitt, einen ersten und einen zweiten Anlageabschnitt sowie eine ersten und eine zweite Luftdurchlassfläche aufweist. Das Filterschutzhalstuch ist vorzugsweise als Dreieckstuch ausgebildet, ohne auf diese Form beschränkt zu sein. Das Filterschutzhalstuch ist aus einem flexiblen Gewebe beschaffen, wobei es sich um jedes geeignete textile oder sonstige Gewebe handeln kann. Dieses wird nachfolgend auch als Gewebe bezeichnet.

Es liegen ferner insbesondere die nachfolgenden Begriffsbestimmungen zu Grunde.

Der Träger des Filterschutzhalstuchs wird nachfolgend auch als Anwender bezeichnet. Die Richtungs- und Lageangabe innen bezeichnet die Seite oder Fläche, die bei bestimmungsgemäßem Tragen des Filterschutzhalstuchs der Halsregion des Anwenders zugewandt ist oder dort aufliegt. Die Richtungs- und Lageangabe außen bezeichnet die die Seite oder Fläche, die innen gegenüberliegt und von der Halsregion weg weist. Die Richtungs- und Lageangabe proximal bezeichnet die zur Mitte der Fläche des Filterschutzhalstuchs weisende Richtung und die Richtungs- und Lageangabe distal die zu proximal entgegengesetzte Richtung zu den Enden des Filterschutzhalstuchs, die bei bestimmungsgemäßen Tragen durch den Anwender vorzugsweise am Nacken des Anwenders positioniert sind.

Der Überdeckungsabschnitt ist an der Halstuchvorderseite und hierbei vorzugsweise in der Mitte des Filterschutzhalstuchs angeordnet. Als Halstuchvorderseite wird der Bereich des Filterschutzhalstuchs verstanden, der bei einem bestimmungsgemäßen Tragen des Filterschutzhalstuchs an der Halsvorderseite des Anwenders angeordnet ist und damit dem Nackenbereich gegenüber liegt.

Der Überdeckungsabschnitt weist eine nach außen gerichtete Ausformung auf. Besonders bevorzugt handelt es sich bei der Ausformung um eine einfache Wölbung des flexiblen Gewebes. Gegenüberliegend auf der Innenseite bildet diese Ausformung den Aufnahmeraum aus. Der Aufnahmeraum wird somit durch eine innenseitig betrachtet konkave und in horizontaler Richtung längserstreckte Mulde ausgeformt, deren Form und Größe auf einen Tracheostomafilter abgestimmt ist. Bei einem bestimmungsgemäßen Tragen ist das Filterschutzhalstuch so am Hals des Anwenders positioniert, dass der Tracheostomafilter in dem Aufnahmeraum angeordnet und nach oben, vorne und unten durch die Innenseite der Ausformung umgeben wird, ohne dass das Filterschutzhalstuch drückt oder in sonstiger Weise eine Kraft auf den Tracheostomafilter einwirkt.

Der Überdeckungsabschnitt weist beidseits distal Ränder auf, bei denen es sich um den ersten und den zweiten Überdeckungsabschnittsrand handelt. Vorzugsweise handelt es sich hierbei um umsäumte Gewebekanten. Vorzugsweise führt die Ausformung beidseits bis an die Überdeckungsabschnittsränder heran, so dass die

Überdeckungsabschnittsränder ebenfalls eine bei außenseitiger Betrachtung konvexe Kontur aufweisen.

Die Anlageabschnitte sind beidseits des Überdeckungsabschnitts angeordnet und vorzugsweise in gleicher Weise sowie symmetrisch ausgebildet, so dass die Beschreibungsinhalte auf beide Anlageabschnitte in entsprechender Weise zugleich zutreffen.

An dem proximalen Anlageabschnittsende, das dem Überdeckungsabschnitt zugewandt ist, ist der jeweilige Anlageabschnittsrand angeordnet. Auch bei dem Anlageabschnittsrand handelt es sich um eine vorzugsweise umsäumte Gewebekante. An dem distalen Anlageabschnittende ist jeweils ein Verschlussmittel angeordnet. Bei dem Verschlussmittel kann es sich insbesondere um einen Knopf, Druckknopf oder eine Klettverschlussfläche beziehungsweise um das jeweilige korrespondierende Gegenstück wie ein Knopfloch und so weiter handeln. Die Verschlussmittel bilden zusammen den Verschluss aus und sichern somit die Lage des Filterschutzhalstuchs am Hals des Anwenders.

Im Gegensatz zu dem Überdeckungsabschnitt sind die Anlageabschnitte außerhalb des Bereichs des Tracheostomafilters angeordnet, so dass die Anlageabschnitte bei bestimmungsgemäßem Tragen mit deren jeweiliger Innenseite praktisch vollflächig an dem Hals des Anwenders anliegen. Dies trifft auch auf die Anlageabschnittsränder zu, so dass diese im Wesentlichen geradlinig vertikal verlaufen.

Durch den einerseits geradlinigen Verlauf der Anlageabschnittsränder und der andererseits konkaven Kontur der Überdeckungsabschnittsränder wird jeweils zwischen dem Anlageabschnittsrand und dem Überdeckungsabschnittsrand eine Fläche aufgespannt. Hierbei handelt es sich um die beiden Luftdurchlassflächen. Aufgrund der Lage des Tracheostomafilters am Hals des Anwenders und der Form der Ausformung und der Position des Aufnahmeraums sind die beiden Luftdurchlassflächen bei einem bestimmungsgemäßen Tragen des Filterschutzhalstuchs durch den Anwender jeweils vor den seitlichen Öffnungen des Tracheostomafilters angeordnet. Die Lagebeziehung der beiden Luftdurchlassflächen lässt sich somit auch so bezeichnen, dass diese den Aufnahmeraum jeweils lateral begrenzen. Vorzugsweise ist dort ein grobmaschiges Gewebe eingenäht, das einen ausreichenden Luftdurchlass ermöglicht.

Das erfindungsgemäße Filterschutzhalstuch weist insbesondere die nachfolgend beschriebenen besonderen Vorteile auf.

Das Filterschutzhalstuch bewirkt einen zusätzlichen mechanischen Schutz des Tracheostomafilters. Dies ist sehr vorteilhaft, da stets die Gefahr besteht, dass sich beispielsweise Schlaufen, Kleidungsstücke oder Ähnliches im Alltag oder beim An- und Auskleiden verfangen können. Weiterhin wird durch die Umfassung des Tracheostomafilters durch den Überdeckungsabschnitt der Tracheostomafilter stärker in die Wärmeeinflusszone des Anwenders gebracht, so dass dieser neben der Temperierung der Ausatemluft zusätzlich durch die Temperatur an der Körperoberfläche des Anwenders erwärmt wird. Insbesondere bei ungünstigen Witterungsbedingungen wie Kalt-, Schlag- oder Pressluft wird somit ein Außenaufenthalt für den Anwender wesentlich erleichtert oder erst ermöglicht.

Bei der Anwendung bei kleinen Kinder sichert das Filterschutzhalstuch zudem den Tracheostomafilter besser vor einem bewussten oder unbewussten Zugriff durch das Kind und schützt somit zudem vor möglichen Verletzungen im Tracheostomabereich.

Das Filterschutzhalstuch ist durch den Anwender einfach anzulegen. Es verdeckt den Tracheostomafilter, so dass dieser nicht oder lediglich dezent wahrgenommen wird.

Gemäß einer ersten vorteilhaften Weiterbildung ist das Filterschutzhalstuch dadurch gekennzeichnet, dass die Luftdurchlassflächen jeweils ein luftdurchlässiges Gewebe aufweisen. Das luftdurchlässige Gewebe ist an der ersten Luftdurchlassfläche mit den beiden ersten Abschnittsrändern und an der zweiten Luftdurchlassfläche mit den beiden zweiten Abschnittsrändern verbunden.

Bei dem luftdurchlässigen Gewebe kann es sich vorzugsweise um ein gelöchertes Leichttextil handeln, das an den Abschnittsrändern eingenäht ist und nachfolgend auch als Air Mesh bezeichnet wird. Das Air Mesh schützt vorteilhaft die Luftdurchlassflächen vor dem Eindringen von Gegenständen und kann eine grobe Vorfilterung der Einatemluft bereitstellen. Zudem kann die Sicherung der Lagebeziehung der Abschnittsränder unterstützt und somit ein ausreichender Querschnitt der Luftdurchlassflächen offen gehalten werden. Weiterhin schützt das Air Mesh davor, dass sich Kleidungsteile oder andere Gegenstände an den Abschnitträndern verfangen.

Entsprechend einer nächsten vorteilhaften Weiterbildung ist das Filterschutzhalstuch dadurch gekennzeichnet, dass jeweils den Luftdurchlassflächen oder der Ausformung eine Stützstruktur zugeordnet ist.

Bei einer solchen Stützstruktur kann es sich beispielsweise um aussteifende oder elastische Elemente handeln, die in die Abschnittsränder oder in den Bereich der Ausformung eingenäht sind und bewirken, dass die Luftdurchlassflächen einen ausreichenden Öffnungsquerschnitt aufweisen und sich auch bei äußeren Einwirkungen, wie beispielsweise durch Kleidungsstücke, nicht zusammendrücken, oder dass der Aufnahmeraum aufgespannt bleibt und keine Kräfte auf den Tracheostomafilter einwirken. Beispielsweise kann der Aufnahmeraum durch eine in die Ausformung eingelassene Kunststoffhalbschale geschützt und zugleich der Bereich der Luftdurchlässe so geformt werden, dass deren Querschnitt stets geöffnet ist.

Die Erfindung wird als Ausführungsbeispiel anhand von
- Fig. 1: Schematische Darstellung in einer Schrägansicht
- Fig. 2: Schematische Darstellung in einer Seitenansicht
näher erläutert.

Hierbei beziehen sich gleiche Bezugszeichen in den verschiedenen Figuren auf jeweils gleiche Merkmale oder Bauteile. Die Bezugszeichen werden in der Beschreibung auch dann verwandt, sofern sie in der betreffenden Figur nicht dargestellt sind.

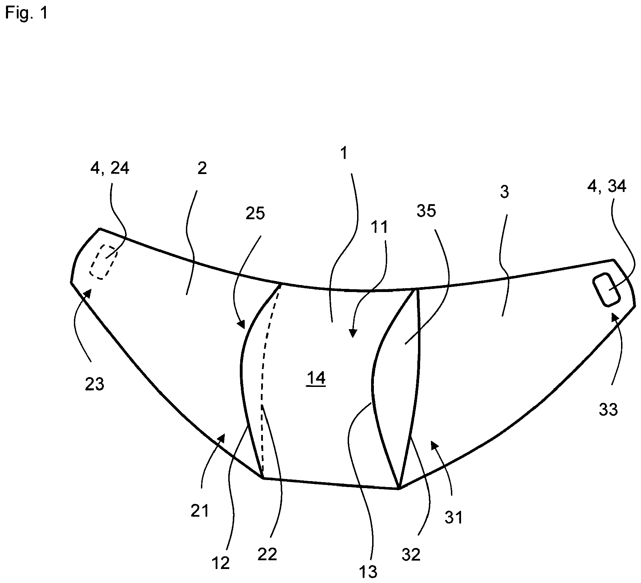

Fig. 1 zeigt ein Ausführungsbeispiel, bei dem der Überdeckungsabschnitt 1 und der erste Anlageabschnitt 2 und der zweite Anlageabschnitt 3 auf einem Jersey-Stoff ausgebildet sind.

Der Überdeckungsabschnitt 1 weist eine Ausformung 11 auf, die im Ausführungsbeispiel nahezu dessen gesamte Fläche erfasst und die seitlich bis an den vertikalen ersten Überdeckungsabschnittsrand 12 und auf der anderen Seite bis an den vertikalen zweiten Überdeckungsabschnittsrand 13 führt. Die Ausformung 11 wird durch eine nach außen konkave Stoffwölbung gebildet, so dass damit auf der Rückseite, die als Innenseite zum Hals des Anwenders zeigt, ein Aufnahmeraum 14 geformt wird. Dort ist beim Tragen des Filterschutzhalstuchs durch den Anwender der Tracheostomafilter - in Fig. 1 nicht dargestellt - angeordnet, der auf diese Weise von dem Filterschutzhalstuch umgeben und geschützt wird.

Der erste Anlageabschnitt 2 besteht im vorliegenden Ausführungsbeispiel aus dem gleichen Material wie der Überdeckungsabschnitt 1 und weist an einem proximalen ersten Anlageabschnittsende 21 einen ersten Anlageabschnittsrand 22 auf, der dem nach außen gewölbten vertikalen ersten Überdeckungsabschnittsrand 12 gegenüberliegt. In die zwischen den beiden ersten Abschnittsrändern 12, 22 somit gebildete erste Luftdurchlassfläche 25 ist ein Air Mesh - Textil eingenäht, wobei hiermit zugleich die Stoffränder der beiden ersten Abschnittsränder 12, 22 umsäumt werden. An dem distalen ersten Anlageabschnittsende 23 ist als erstes Verschlussmittel 24 innenseitig ein Klettverschluss eingenäht.

Der zweite Anlageabschnitt 3 ist gegenüberliegend zu dem ersten Anlageabschnitt 2 angeordnet und weist mit proximalem zweiten Anlageabschnittende 31 und zweitem Anlageabschnittsrand 32 sowie distalem zweiten Anlageabschnittsende 33 und hier außenseitig angebrachtem zweiten Verschlussmittel 34 einen entsprechend Aufbau wie der erste Anlageabschnitt 2 auf, so dass über die beiden zweiten Abschnittränder 13, 32 die zweite Luftdurchlassfläche 35 ebenfalls in entsprechender Weise zu der ersten Luftdurchlassfläche 25 gebildet werden, so dass die jeweiligen Beschreibungsinhalte zu dem ersten Anlageabschnitt 2 und zu der ersten Luftdurchlassfläche 25 hier in entsprechender Weise gelten.

Die beiden Luftdurchlassflächen 25, 35 begrenzen den Aufnahmeraum 14 jeweils seitlich und ermöglichen an den dort befindlichen Öffnungen des Tracheostomafilters einen weitgehend ungehinderten Luftstrom. Hierfür sind im vorliegenden Ausführungsbeispiel die Abschnittränder 12, 22, 13, 32 ausgesteift, so dass die Luftdurchlassflächen 25, 34 stets offen gehalten werden.

Um einen sicheren Halt des Filterschutzhalstuchs zu ermöglichen, werden nackenseitig die beiden Verschlussmittel 24, 34, hier vorliegend als zwei Klettverschlussflächen, zu einem Verschluss 4 zusammengeführt.

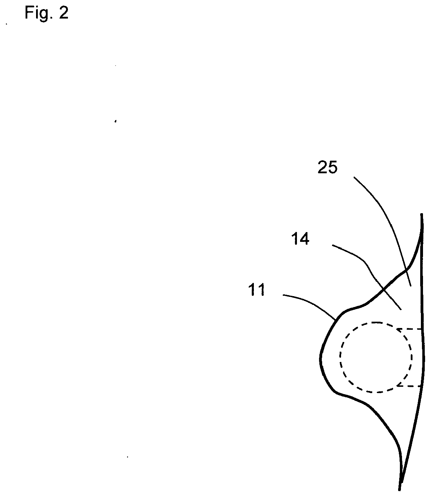

Ferner zeigt die Fig. 2 in einer schematischen Seitenansicht von links die Lagebeziehung des Filterschutzhalstuchs zu dem hier mit einer Strichlinie und ohne Bezugszeichen dargestellten Tracheostomafilter bei bestimmungsgemäßer Verwendung. Die Ausformung 11 bildet innenseitig den Aufnahmeraum 14 aus, der lateral durch die Luftdurchlassflächen 25, 35 begrenzt wird, wobei in der Darstellung von Fig. 2 lediglich die erste Luftdurchlassfläche 25 zu sehen ist, während die zweite Luftdurchlassfläche 35 gegenüberliegend verdeckt angeordnet ist. Die beiden seitlichen Öffnungen des Tracheostomafilters sind jeweils in Richtung der Luftdurchlassflächen 25, 35 gerichtet.

### Verwendete Bezugszeichen

- 1: Überdeckungsabschnitt
- 11: Ausformung
- 12: erster Überdeckungsabschnittsrand
- 13: zweiter Überdeckungsabschnittsrand
- 14: Aufnahmeraum
- 2: erster Anlageabschnitt
- 21: proximales erstes Anlageabschnittsende
- 22: erster Anlageabschnittsrand
- 23: distales erstes Anlageabschnittsende
- 24: erstes Verschlussmittel
- 25: erste Luftdurchlassfläche
- 3: zweiter Anlageabschnitt
- 31: proximales zweites Anlageabschnittsende
- 32: zweiter Anlageabschnittsrand
- 33: distales zweites Anlageabschnittsende
- 34: zweites Verschlussmittel
- 35: zweite Luftdurchlassfläche
- 4: Verschluss

## Patentansprüche

1. Filterschutzhalstuch,
aufweisend einen Überdeckungsabschnitt (1), einen ersten Anlageabschnitt (2), eine erste Luftdurchlassfläche (25), einen zweiten Anlageabschnitt (3) und eine zweite Luftdurchlassfläche (35)
wobei der Überdeckungsabschnitt (1) an einer Halstuchvorderseite angeordnet ist, eine außenseitig gerichtete Ausformung (11) aufweist und innenseitig einen Filteraufnahmeraum (4) ausbildet, wobei die Ausformung (11) zur außenseitigen Überdeckung und der Filteraufnahmeraum (14) zur Aufnahme eines Tracheostomafilters ausgebildet ist
wobei der Überdeckungsabschnitt (1) jeweils distal einen vertikalen ersten und einen zweiten Überdeckungsabschnittsrand (12, 13) aufweist,
wobei der erste Anlageabschnitt (2) distal zu dem Überdeckungsabschnitt (1) angeordnet und innenseitig für eine flächige Anlage am Hals eines Anwenders ausgebildet ist sowie an einem proximalen ersten Anlageabschnittsende (21) einen ersten Anlageabschnittsrand (22) und an einem distalen ersten Anlageabschnittsende (23) ein erstes Verschlussmittel (24) aufweist,
wobei die erste Luftdurchlassfläche (25) durch eine zwischen dem ersten Überdeckungsabschnittsrand (12) und dem ersten Anlageabschnittsrand (22) aufgespannte Fläche gebildet ist,
wobei der zweite Anlageabschnitt (3) distal zu dem Überdeckungsabschnitt (1) angeordnet und innenseitig für eine flächige Anlage am Hals eines Anwenders ausgebildet ist sowie an einem proximalen zweiten Anlageabschnittsende (31) einen zweiten Anlageabschnittsrand (32) und an einem distalen zweiten Anlageabschnittsende (33) ein zweites Verschlussmittel (34) aufweist, wobei die Verschlussmittel (24, 34) zusammen einen nackenseitigen Verschluss (4) ausbilden,
wobei die zweite Luftdurchlassfläche (35) durch eine zwischen dem zweiten Überdeckungsabschnittsrand (13) und dem zweiten Anlageabschnittsrand (32) aufgespannte Fläche gebildet ist.

2. Filterschutzhalstuch nach Anspruch 1,
**dadurch gekennzeichnet,**
**dass** die Luftdurchlassflächen (25, 35) jeweils ein luftdurchlässiges Gewebe aufweisen
das an der ersten Luftdurchlassfläche (25) mit den beiden ersten Abschnitträndern (12, 22) und an der zweiten Luftdurchlassfläche (35) mit den beiden zweiten Abschnitträndern (13, 32) verbunden ist.

3. Filterschutzhalstuch nach einem der vorhergehenden Ansprüche,
**dadurch gekennzeichnet,**
**dass** jeweils den Luftdurchlassflächen (25, 35) oder der Ausformung (11) eine Stützstruktur zugeordnet ist.
